# EUROPÄISCHE PATENTANMELDUNG

(11) **EP 1 930 311 A1**
(43) Veröffentlichungstag der Anmeldung: **11.06.2008**
(21) Anmeldenummer: 06025406.7
(22) Anmeldetag: 08.12.2006
(51) Int. Cl.: C07C 69/96, A61K 8/37

(54) **Dialkylcarbonate von verzweigten Alkoholen und ihre Verwendung**

(71) Anmelder: Cognis IP Management GmbH, 40589 Düsseldorf (DE)
(72) Erfinder: Ansmann, Achim, 40699, Erkrath (DE); Boutty, Bernd, 40667 Meerbusch (DE); Dierker, Markus, 40597 Düsseldorf (DE)

(57) **Zusammenfassung**

Vorgeschlagen werden Dialkylcarbonate von verzweigten Alkoholen sowie ihre Verwendung in kosmetischen Zubereitungen.

## Beschreibung

### Gebiet der Erfindung

Die Erfindung betrifft Dialkylcarbonate von verzweigten Alkoholen und ihre Verwendung in kosmetischen und/oder pharmazeutischen Zubereitungen.

### Stand der Technik

Im Bereich kosmetischer Zubereitungen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet.

Dialkylcarbonate von linearen Alkoholen und ihre kosmetische Verwendungsind aus dem Stand der Technik bekannt, so beschreibt WO 97147282 die kosmetische Verwendung von Octyl-methyl-carbonat, EP 1 510 199 die kosmetische Verwendung von Distearylcarbonat. Di-n-octylcarbonat ist als kosmetischer Rohstoff unter dem Handelsnamen Cetiol® CC (Cognis Deutschland GmbH & Co. KG) erhältlich.

Die Herstellung von Dialkylcarbonaten ist in WO 97/47583 beschrieben. Die nach dem dort beschriebenen Verfahren erhältlichen symmetrischen Dialkylcarbonate enthalten als Nebenprodukte z.B. 2-Ethyl-1-hexyl-methylcarbonat oder 2-Butyl-1-Octyl-methylcarbonat. Eine kosmetische Verwendung dieser Verbindungen wird nicht erwähnt. EP 1 083 247 beschreibt verschiedene kurzkettige verzweigte Dialkylcarbonate und ihre Eignung zum Waschen von Metalloberflächen.

Aufgabe der vorliegenden Erfindung war es, neue Rohstoffe für kosmetische Applikationen zur Verfügung zu stellen, die bezüglich ihrer sensorischen Eigenschaften (Leichtigkeit, "nicht-fettendes Hautgefühl", Weichheit, Spreitvermögen, Absorption, Verteilbarkeit, Öligkeit) über ein verbessertes Profil verfügen und in eine Vielzahl kosmetischer Formulierungen einarbeitbar sind, beispielsweise als Ölkörper/Emollient oder als Konsistenzgeber. Die Rohstoffe sollten sowohl in W/O als auch in O/W Formulierungen einarbeitbar sein und insbesondere mit kristallinen UV-Filtem, Pigmenten, Antiperspirantien Salzen sowie Silikonen kompatibel sein.

Überraschenderweise wurde gefunden, dass die Dialkylcarbonate der vorliegenden Erfindung diese Aufgabe erfüllen und insbesondere zu sensorisch leichten Produkten führen.

### Beschreibung der Erfindung

Dialkylcarbonate sind Ester der Kohlensäure der allgemeinen Formel O=C(OR)₂, wobei R einen AlkylRest bedeutet. Die Bezeichnung der Dialkylcarbonate erfolgt entweder, indem die Alkylsubstituenten dem Begriff "Carbonat" voran stellt oder als Kohlensäure"alkyl"ester. So z.B. O=C(OC₈H₁₇)₂ als Dioctylcarbonat oder Kohlensäuredioctylester oder z.B. O=C(OC₂H₅)(OC₈H₁₇) als Ethyl-octyl-carbonate oder als Kohlensäure-Ethyl-Octylester. Im Folgenden wird für die Dialkylcarbonate die Formel R-O-CO-O-R verwendet.

Gegenstand der Erfindung sind Dialkylcarbonate der Formel (I)

**R¹**O-CO-OR**²** **(I)**

wobei R¹ ein 2- Propyl-1-heptyl-Rest oder ein 2-Ethyl-1-Butyl Rest ist, und R² für lineare oder verzweigte oder cyclische Kohlenwasserstoffreste mit 1 bis 22 Kohlenstoffatomen und 0 oder 1 bis 3 Doppelbindungen steht.

In einer bevorzugten Ausführungsform der Erfindung ist R¹ identisch zu R²: Diese Ausführungsform der Erfindung betriff somit Di-(2- Propyl-1-heptyl)carbonat bzw. Di-(2-Ethyl-1-Butyl)carbonat.

Ein weiterer Gegenstand der Erfindung betrifft Dialkylcarbonate der Formel (11)

**R³O-CO-OR⁴** **(II)**

wobei R³ ausgewählt ist aus der Gruppe bestehend aus Isodecyl-Rest, Neodecyl-Rest, Isoundecyl-Rest, Isododecyl-Rest, Isotridecyl-Rest, 2-Pentylnonyl-Rest, 2-Hexyldecyl-Rest, 2-Heptyl-undecylrest, Isostearyl-Rest, 2-Octyl-dodecyl-Rest, 2-Butyl-1-octyl-Rest und 2-Ethyl-1-hexyl Rest, und R⁴ für lineare oder verzweigte oder cyclische Kohlenwasserstoffreste mit 2 bis 22 Kohlenstoffatomen und 0 oder 1 bis 3 Doppelbindungen steht, wobei R³ nicht identisch ist zu R⁴.

Ein weiterer Gegenstand der Erfindung betrifft Dialkylcarbonate der Formel (III)

**R⁵**O-CO-OR**⁶** **(III)**

in der R⁵ für lineare oder verzweigte oder cyclische Kohlenwasserstoffreste mit 1 bis 22 Kohlenstoffatomen und 0 oder 1 bis 3 Doppelbindungen steht und R⁶ für verzweigte Kohlenwasserstoffreste mit mehr als 12 Kohlenstoffatomen und 0 oder 1 bis 3 Doppelbindungen.

Ein weiterer Gegenstand der Erfindung betrifft die Verwendung von Dialkylcarbonaten der Formel (IV)

**R⁷**O-CO-OR**⁸** **(IV)**

in der R⁷ für verzweigte Kohlenwasserstoffreste mit 3 bis 22 Kohlenstoffatomen und 0 oder 1 bis 3 Doppelbindungen steht und R⁸ für lineare oder verzweigte oder cyclische Kohlenwasserstoffreste mit 1 bis 22 Kohlenstoffatomen und 0 oder 1 bis 3 Doppelbindungen steht und wobei R⁷ nicht identisch zu R⁸ ist, in kosmetischen Zubereitungen. In einer bevorzugten Ausführungsform der Erfindung werden Dialkylcarbonate der Formel (IV) verwendet, bei denen R⁸ für verzweigte Kohlenwasserstoffreste mit 3 bis 22 Kohlenstoffatomen und 0 oder 1 bis 3 Doppelbindungen steht.

In einer besonders bevorzugten Ausführungsform der Erfindung werden Dialkylcarbonate der Formel (IV) verwendet, welche eine Gesamtkohlenstoffzahl von größer 12 enthalten.

R², R⁵ sowie R⁸ kann ein lineare oder verzweigte oder cyclische Kohlenwasserstoffrest mit 1 bis 22 Kohlenstoffatomen und 0 oder 1 bis 3 Doppelbindungen sein, R⁴ kann ein linearer oder verzweigter oder cyclischer Kohlenwasserstoffreste mit 2 bis 22 Kohlenstoffatomen und 0 oder 1 bis 3 Doppelbindungen sein. R⁷ kann ein verzweigter Kohlenwasserstoffreste mit 3 bis 22 Kohlenstoffatomen und 0 oder 1 bis 3 Doppelbindungen sein:

Beispielsweise seien als lineare Alkylreste genannt: Methyl-, Ethyl-, Butyl-, Pentyl-, Hexyl-, Heptyl-, Octyl-, Nonyl-, Decyl-, Undecyl-, Undecenyl-, Dodecyl- , Tridecyl-, Tetradecyl-, Pentadecyl-, Hexadecyl-, Heptadecyl-, Octadecyl-, Octadecenyl-, Nonadecyl-, Eicosanyl (=C20)-, Docosanyl (= C22)- Reste genannt.

Beispielsweise seien als verzweigte Alkylreste genannt: 2-Methylpropyl-, iso-Butyl-, iso-Pentyl, wie z.B. 2,2,-Dimethylpropyl (=Neopentyl), 3-Methylbutyl- (=iso-Pentyl-), iso-Hexyl-, i-Octyl, wie z.B. 2-Ethyl-hexyl- oder 3-Ethyl-hexyl oder 4-Ethylhexyl- oder 5-Ethylhexyl Reste, i-Decyl-Reste wie z.B. Trimethylheptyl-Rest (= Neodecyl-Rest), Isostearyl-, Isooctyl-, Isononyl-, Isodecyl-, Isotridecyl-, 2-Ethylbutyl-, 2-Ethylhexyl-, 2-Propylheptyl, 2-Butyloctyl, 2-Butyldecyl-, 2-Hexyloctyl-, 2-Hexyldecyl-, 2-Hexyldodecyl-, 2-Octyldecyl-Rest.

Als cyclische Alkyreste seinen genannt der Bomeyl- und Isobomeyl-Rest sowie Cyclohexyl-Rest.

Ein weiterer Gegenstand der Erfindung betrifft die Verwendung aller vorgenannten Dialkylcarbonate in kosmetischen Zubereitungen. Überraschenderweise wurde festgestellt, dass sich die erfindungsgemäßen Dialkylcarbonate besonders eignen zur Herstellung von kosmetischen Zubereitungen, sie eignen sich insbesondere als Ölkörper/Emollients und/oder Konsistenzgeber in kosmetischen Zubereitungen. Die erfindungsgemäßen Dialkylcarbonate eignen sich weiterhin zur Herstellung von pharmazeutischen Zubereitungen, wobei die Dialkylcarbonate als technische Hilfsstoffe, wie z.B. Ölkörper eingesetzt werden. Die erfindungsgemäßen Dialkylcarbonate können zur Herstellung von kosmetischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudem oder Salben dienen. Diese Mittel können ferner als weitere Hilfs- und Zusatzstoffe milde Tenside, Ölkörper, Emulgatoren, Perlglanzwachse, Konsistenzgeber, Verdickungsmittel, Überfettungsmiftel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Bevorzugt ist die Verwendung der Dialkylcarbonate als Ölkörper.

Die erfindungsgemäßen Dialkylcarbonate können in kosmetischen Formulierungen als sog., light emollients' verwenden werden, um spezielle Eigenschaften, wie z.B. Spreitverhalten oder Flüchtigkeit einzustellen. Die erfindungsgemäßen Dialkylcarbonate ermöglichen weiterhin viskositätsstabile kosmetische Formulierungen herzustellen.

### Herstellung

Die Herstellung der Dialkylcarbonate erfolgt nach dem Fachmann bekannten Verfahren beispielsweise durch Umesterung niederer Dialkylcarbonate, wie beispielsweise Dimethylcarbonat, Diethylcarbonat, Dipropylcarbonat oder Dibutylcarbonat mit den entsprechenden Alkoholen oder Alkoholgemischen in Gegenwart eines Katalysators, wie beispielsweise in WO 97/47583 beschrieben.

Andere Herstellverfahren sind beschrieben in M. Dierker: Lipid Technology, 2004,16(6), S. 130-134**.**

### Beispiele

### Beispiel 1: Herstellung von Hexyldecyl-methylcarbonat

1350g (15 mol) Dimethylcarbonat wurden mit 14g Natriummethylat (30%ig in Methanol) in einer 4 I Rührapparatur mit Tropftrichter vorgelegt und auf 80°C aufgeheizt. Dann wurde 699g (1,5 mol) Hexyldecanol über einen Zeitraum von 2 Stunden zugetropft und weitere 9 Stunden 80°C nachgerührt. Entstehendes Methanol wurde währenddessen über einen Destillationsaufsatz aus der Reaktionsmischung abdestilliert. Die GC Auswertung des Rohproduktes ergab ein Zusammensetzung von: 4% Hexyldecanol, 79% asymmetrisches Hexyldecyl-methyl-carbonat und 13% symmetrisches Di-Hexyldecylcarbonat.
Der pH-Wert wurde mit 14g H₃PO₄ (85%ig) auf pH 3-4 eingestellt und überschüssiges Dimethylcarbonat abdestilliert. Das ausgefallene Natriumphosphat wurde abgesaugt und das Produkt fraktioniert. Das Reaktionsprodukt Hexyldecyl-methyl-carbonat fällt als farbloses Öl mit einem Siedepunkt von 125-125°C bei 0,4 mbar an.

### Beispiel 2: Formulierungen mit Hexyldecyl-methylcarbonat

Die nachfolgenden kosmetischen Rezepturen wurden mit dem nach Beispiel 1 hergestellten Hexyldecyl-methylcarbonat hergestellt. Alle Angaben sind in Gew.-%

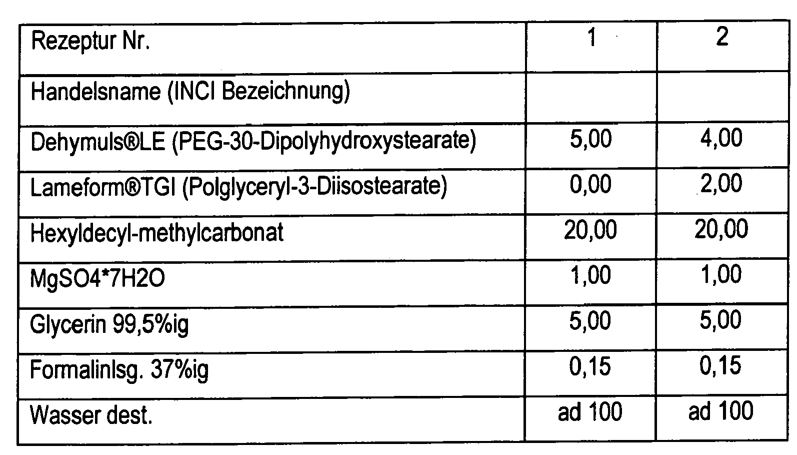

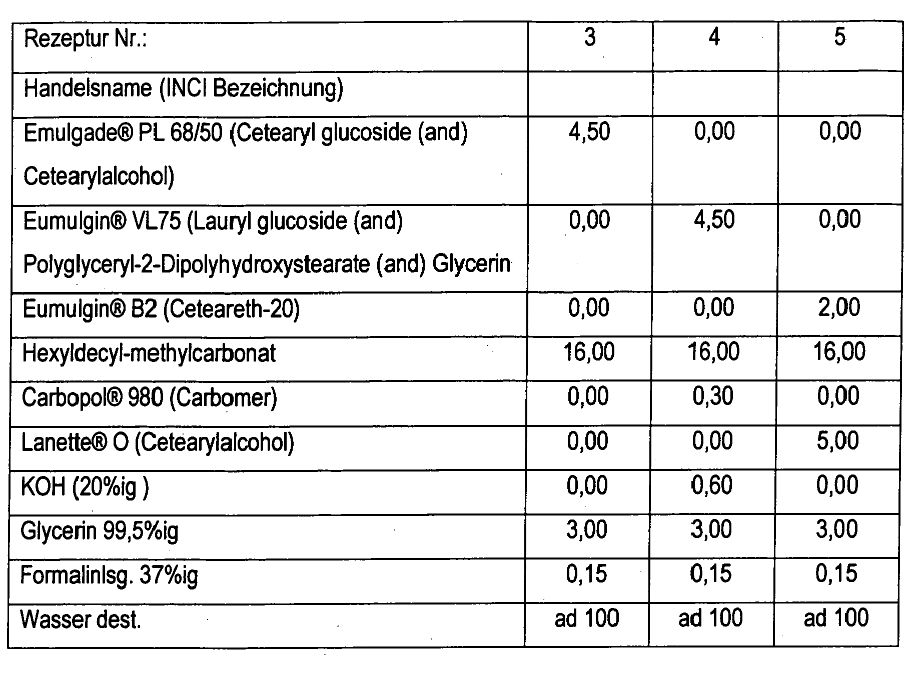

## Patentansprüche

1. Dialkylcarbonate der Formel (I) **R¹O-CO-OR² (I)**
wobei R¹ ein 2- Propyl-1-heptyl-Rest oder ein 2-Ethyl-1-Butyl Rest ist, und R² für lineare oder verzweigte oder cyclische Kohlenwasserstoffreste mit 1 bis 22 Kohlenstoffatomen und 0 oder 1 bis 3 Doppelbindungen steht.

2. Dialkylcarbonate nach Anspruch 1, wobei R² identisch ist zu R¹.

3. Dialkylcarbonate der Formel (II) **R³O-CO-OR⁴ (II)**
wobei R³ ausgewählt ist aus der Gruppe bestehend aus Isodecyl-Rest , Neodecyl-Rest, Isoundecyl-Rest, Isododecyl-Rest, Isotridecyl-Rest, 2-Pentylnonyl-Rest, 2-Hexyldecyl-Rest, 2-Heptyl-undecylrest, Isostearyl-Rest, 2-Octyl-dodecyl-Rest, 2-Butyl-1-octyl-Rest und 2-Ethyl-1-hexyl Rest, und R⁴ für lineare oder verzweigte oder cyclische Kohlenwasserstoffreste mit 2 bis 22 Kohlenstoffatomen und 0 oder 1 bis 3 Doppelbindungen steht, wobei R³ nicht identisch ist zu R⁴.

4. Dialkylcarbonate der Formel (III) **R⁵O-CO-OR⁶ (III)**
in der R⁵ für lineare oder verzweigte oder cyclische Kohlenwasserstoffreste mit 1 bis 22 Kohlenstoffatomen und 0 oder 1 bis 3 Doppelbindungen steht und R6 für verzweigte Kohlenwasserstoffreste mit mehr als 12 Kohlenstoffatomen und 0 oder 1 bis 3 Doppelbindungen.

5. Verwendung der Dialkylcarbonate nach mindestens einem der Ansprüche 1 bis 4 in kosmetischen Zubereitungen.

6. Verwendung von Dialkylcarbonaten der Formel (IV) **R⁷O-CO-OR⁸ (IV)**
in der R⁷ für verzweigte Kohlenwasserstoffreste mit 3 bis 22 Kohlenstoffatomen und 0 oder 1 bis 3 Doppelbindungen steht und R⁸ für lineare oder verzweigte oder cyclische Kohlenwasserstoffreste mit 1 bis 22 Kohlenstoffatomen und 0 oder 1 bis 3 Doppelbindungen steht und wobei R⁷ nicht identisch zu R⁸ ist, in kosmetischen Zubereitungen.

7. Verwendung nach Anspruch 6, **dadurch gekennzeichnet, dass** R⁸ für verzweigte Kohlenwasserstoffreste mit 3 bis 22 Kohlenstoffatomen und 0 oder 1 bis 3 Doppelbindungen steht.

8. Verwendung nach Anspruch 6 oder 7, **dadurch gekennzeichnet, dass** die Dialkylcarbonate mehr als 12 Kohlenstoffatome enthalten.
